Europäisches Patentamt

European Patent Office

Office européen des brevets

(19)

(11) Publication number: **0 009 277**
**B1**

(12) **EUROPEAN PATENT SPECIFICATION**

(45) Date of publication of patent specification: **18.04.84**

(21) Application number: **79200490.5**

(22) Date of filing: **05.09.79**

(51) Int. Cl.³: **A 61 K 39/295,**
**C 12 N 7/00** //A61K39/17,
A61K39/235

(54) Combined vaccine active against Newcastle disease and egg production drop caused by adeno-like viruses, process for preparing it, adeno-like and Newcastle disease virus strains.

(30) Priority: **14.09.78 NL 7809364**
**26.04.79 NL 7903300**

(43) Date of publication of application:
**02.04.80 Bulletin 80/7**

(45) Publication of the grant of the patent:
**18.04.84 Bulletin 84/16**

(84) Designated Contracting States:
**AT CH DE GB IT NL SE**

(56) References cited:
**LU - A - 79 154**

**CHEMICAL ABSTRACTS, vol. 89, no. 13, 25th**
**September 1978, page 422, no. 103639g**
**Columbus, Ohio, U.S.A. D. TODD ét al.:**
**"Biochemical studies on a virus associated with**
**egg drop syndrome 1976"**
**UNILISTED DRUGS, vol. 14, no. 10, October**
**1962, page 98**

**The file contains technical information**
**submitted after the application was filed and not**
**included in this specification**

(73) Proprietor: **Gist - Brocades N.V.**
**Wateringseweg 1 P.O. Box 1**
**NL-2600 MA Delft (NL)**

(72) Inventor: **Apontoweil, Peter**
**Burg. Martenslaan 39**
**NL-3956 EK Leersum (NL)**
Inventor: **Kok, Johannes Anthonius Gerardus**
**Prins Bernhardlaan 10**
**NL-3761 AB Soest (NL)**

(74) Representative: **Van der Straaten, Jan Anthony**
**et al,**
**c/o GIST-BROCADES N.V. Patents and**
**Trademarks Department Wateringseweg 1 P.O.**
**Box 1**
**NL-2600 MA Delft (NL)**

Courier Press, Leamington Spa, England.

# Combined vaccine active against Newcastle disease and egg production drop caused by adeno-like viruses, process for preparing it, adeno-like and Newcastle disease virus strains.

The invention relates to a combined vaccine ready for administration against infections in poultry and more particularly against Newcastle disease and the disease characterized by sudden drop in egg production caused by adeno-like viruses. The invention further relates to the preparation of this combined vaccine and to the preparation process of the vaccine component against the egg production drop.

Newcastle disease [abbreviated ND], one of the most important respiration diseases in poultry, is caused by a virus, and may cause a high mortality in poultry of all ages.

Several vaccines against Newcastle disease have been developed, and it has turned out that those vaccines are preferably administered in their inactivated form, since that form provides a high degree of safety. In addition, the use of a live vaccine sometimes results in the fact that the inoculated virus distributes from vaccinated poultry to poultry susceptible to infections or not being immune.

Although various relatively safe and well immunising live ND vaccines have been developed, as, e.g. appears from British Patent Specification No. 1,510,100, there is still a clear need for inactivated ND vaccines.

Since respiration diseases in poultry are often the result of more than one infection source, combined vaccines have been developed for example that according to U.S. Patent Specification No. 2,798,835, which consists of a combination of a vaccine against infectious bronchitis and an ND vaccine, both components being derived from live viruses.

Furthermore, published Dutch Patent Application No. 7117873 (in the mean time published for opposition purposes under No. 157209) discloses a process for the preparation of a combined vaccine against respiration diseases in poultry, characterized by combination of a dead vaccine against infectious coryza, obtained by cultivating a *Haemophilus gallinarium* strain in a natural nutrient medium and subsequently inactivating the bacteria, with a dead vaccine from an infectious bronchitis virus and a dead ND vaccine.

It is remarked that several references, e.g. Avian Dis. 7 (1963) pages 106 to 122, Am. J. Vet. Res. 17 (1956) pages 294 and 298, Avian Dis. 11 (1967) pages 399 to 406 and Virology 33 (1967) pages 598 to 608, disclose that the activity of each of the components may decrease or may be lost entirely by mixing live viruses, caused by mutual inhibition.

A connection between the presence of adeno-like viruses (hereinafter indicated ALV) and, in part, the sudden drop in egg production and/or increasing production of eggs with soft shells and/or without shells, observed increasingly in the past years, particularly in broiler mother animals and chickens of medium heavy lay races, is already indicated in Avian Pathology 5 (1976) pages 261 to 272. The literature calls such symptoms sometimes "egg drop syndrome". Avian Pathology 6 (1977) pages 405 to 413 also discloses a relation observed between the development of antibodies with regard to a hemagglutinising virus, called 127 virus, and the economically very disadvantageous symptoms described hereinabove. Avian Pathology 7 (1978) pages 35 to 47 confirms the assumed relation between the presence of an adeno-like virus and the sudden drop in production described hereinabove. In addition, several characteristic properties of the type of virus in question are described, e.g. the characteristic agglutination of chicken erythrocytes and the incapability of antisera of neutralising the known adeno virus types. A biochemical study concerning the above-mentioned virus strain 127 is described in J. Chem. Virol (1978) *40*, 63—75.

Luxembourg Patent Specification No. 79154 discloses a process for the preparation of a vaccine against the "egg drop syndrome", starting from an ALV characterized by a specific hemagglutination and antisera reaction. In the description and the examples this virus is called EDS-76 and BC-14. This vaccine is prepared by cultivating the new virus in a tissue culture, preferably in an avian tissue culture such as chicken embryo fibroblasts and embryonal liver or kidney cells or in embryonated duck eggs.

From Avian Pathology 5 (1976) page 262, second paragraph, from Avian Pathology 6 (1977) page 406 and from Luxembourg Patent Specification 79154, page 1, lines 7 to 11 as well, it is evident that an egg production drop may be caused by Newcastle disease, infectious bronchitis, avian and encephalomyelitis, birdpox viruses and combinations thereof.

Therefore, from a practical point of view, poultry will often simultaneously or in close succession be vaccinated with two or more vaccines against the above-mentioned more generally occurring virus diseases. Particularly poultry will simultaneously be vaccinated against Newcastle disease and the production drop caused by ALV as well. The hitherto usual method for any such simultaneous administration consisted of mixing standard volumes of the separate vaccines ready for use and the administration of a vaccine dose composed on the spot, the dose consisting therefore of substantially the double volume as compared to those of the separately administered vaccines, the respective virus amounts being diluted. Moreover, an exact mixing can hardly be guaranteed, while problems due to foreign infectious matter may arise.

In this respect it has to be remarked, however, that a large number of problems will occur in the preparation of combined vaccines, which is emphasized for instance in U.S. patent 3,876,763.

In this patent, which relates to a combined aqueous vaccine containing killed Newcastle disease, killed infectious bronchitis virus and killed Hemophilus gallinarum together with an adjuvant selected from the group consisting of aluminium hydroxide gel, aluminium phosphate gel, calcium phosphate gel and alum, it is said in column 1, lines 59 to 67, that it might be expected that a combination of plural vaccines results in an unfavorable effect on poultry and that a mixture of different vaccines is liable to lose the activities of the individual components through interference or mutual inhibition.

Therefore it is an object of the invention to find a combined, ready for use vaccine, effective against Newcastle disease and the production drop caused by ALV as well, which moreover meets the present severe requirements of the veterinary authorities in most of the relevant countries.

Surprisingly it has been found that a combined, ready for administration vaccine may be prepared by combining an inactivated ND virus liquid and an inactivated ALV liquid.

The invention relates to a hand ready combined inactivated vaccine against Newcastle disease and egg production drop caused by adeno like viruses, characterized in that it contains an aqueous phase, formed by an inactivated Newcastle disease virus containing liquid with a titre of at least $10^{9.8}$ EID 50/ml and an inactivated adeno like virus containing liquid with a titre of at least $10^2$ HA units/ml obtained by cultivation of an in duck's eggs or in duck's embryo fibroblasts passaged virus strain 127 in duck's eggs or in a cell culture of duck's embryo fibroblasts, whereby the volume ratio between the Newcastle disease containing liquid and the adeno like virus containing liquid can vary from 3:4 to 5:1, and an oil phase comprising as essential components at least one of the components selected from the group consisting of light paraffinic mineral oils, vegetable oils and naphthenic mineral oils and at least one emulsifier selected from the group consisting of esters and ether-esters of alkylene oxide adducts and/or hexahydric alcohols with fatty acids of 10—20 carbon atoms whereby the volume ratio between the aqueous phase formed by both virus liquids and the oil phase can vary from 7:13 to 3:7.

It will be appreciated that the invention is also relating to inactivated combination vaccines, derived from at least an NDV liquid and at least an inactivated ALV liquid and optionally derived from more than these two beforementioned virus types.

In the process according to the invention the inactivated NDV-containing liquid is prepared in a manner known per se in principle, e.g. by cultivating the desired virus in fertilized chicken eggs, collecting the cultivated virus in a liquid of a titre usual for this kind of vaccine, inactivating it, suspending the virus in a suitable buffered solution, homogenising it, mixing it with an inactivated virus liquid derived from an ALV, prepared by cultivating the virus in fertilized duck eggs or in a cell culture of duck's cells, collecting the virus produced in a liquid having a titre usual for this kind of vaccines and inactivating the virus liquid according to methods known per se.

For the preparation of the NDV-containing liquid various little virulent or practically avirulent (lentogenic or velogenic) ND viruses having good immunising properties may be used, such as the La Sota or Hitchner virus (lentogenic) or the Herts 33/56 (velogenic) virus.

Specific examples of applicable virus strains, are the strains P/76/5, P/76/4 and P/76/3, obtainable from Institut für Medizinische Mikrobiologie, Infections und Seuchenmedizin der Ludwig Maximilians Universität, München, the VR-108, VR-107, VR-109, VR-699, VR-623 strains, obtainable from the American Type Culture Collection, Rockville, Maryland, USA, the Queensland V 4 strain, obtainable from the Centr. Vet. Labs., Weybridge, Great Britain, or the L/Z 258 P virus, deposited with the Collection d'Institut Pasteur, Paris under I.091.

For the preparation of the inactivated ALV liquid component a passaged virus strain 127, deposited with the Collection d'Institut Pasteur, Paris under no. I.090 is preferably used, although also other, qua antigenicity rather similar, egg drop causing ALV virus types may be successfully applied. This virus strain I.090 is preferably cultivated in duck eggs.

The preparation of the inactivated ALV liquid is preferably started from a virus derived from a passaged virus strain 127 as indicated in the Avian Pathology references cited hereinabove, although other egg production decrease causing ALV types may be applied successfully too.

For the preparation of the combined vaccine 150 to 250 ml of a buffered NDV-containing liquid is inactivated, is optionally homogenised and, together with 100 to 200 ml of a buffered ALV-containing liquid previously inactivated and optionally homogenised, are added to 600 to 700 of an oil phase. The essential components of the oil phase are at least one of the components selected from the group consisting of light paraffinic mineral oils vegetable oils and naphtenic mineral oils and at least one emulsifier selected from the group consisting of ester or ether-esters of alkylene oxide adduct and/or hexahydric alcohols with natural fatty acids of 10—20 carbon atoms.

Examples thereof are mannide monooleate (Span® 80, Arlacel 80®, Arlacel A® and polyoxyethylene (20) sorbitan monooleate (e.g. Tween® 80).

The volume ratio of the buffered NDV-containing liquid and the buffered ALV-containing liquid is preferably 3:2.

The volume ratio of the aqueous phase, formed by both virus liquids, and the oil phase is preferably 7:13.

It has been found that the aqueous phase must be added to the oil phase under vigorous stirring and/or homogenising in order to obtain the desired stable and thin liquid final emulsion.

The oil phase contains 2 to 20% by weight (based on the oil phase weight) of an emulsifier, preferably 2 to 15% by weight of Arlacel A® or Arlacel 80® or Span® 80 and 0.2 to 4% of Tween® 80. The components of the oil phase are preferably separately heated to at least 110°C in an autoclave or sterily filtrated as mixture.

The inactivation of both starting virus liquid components may be performed with the usual inactivators, e.g. by means of $\beta$-propiolactone, optionally combined with a stabiliser, or formaldehyde. Preferably $\beta$-propiolactone is added in a concentration of 0.05 to 0.25% by weight (based on the aqueous phase weight) to a buffered ND virus-containing liquid and the liquid is incubated at about 37°C during 1 to 2 hours, preferably 90 minutes, while the buffered ALV liquid is inactivated by means of formaldehyde and preferably at about 20°C during about 20 hours in a concentration of 0.02 to 0.5% by weight. Then, if necessary, the virus liquids are homogenised in the usual manner. A preservative, such as thiomersal or formaline in a buffered solution, may then optionally be added to the aqueous phase.

In order to obtain the combined vaccine, giving rise to the desired favourable titres after administration, NDV starting solution of a titre of $10^{9.8}$ EID 50/ml to $10^{10.5}$ EID 50/ml and preferably $\geq 10^{10}$ EID 50/ml has to be used and an ALV liquid with an activity of at least $10^2$ HA units, and preferably $10^3$ HA units has to be used.

It will be clear that the favourable properties of the presently proposed combined virus vaccines may not be predicted at all by a person skilled in the art since, due to several undesired reactions, which certainly cannot be excluded by skilled people, the activities of one or both virus components in the final vaccine may decrease to an undesired level, caused by undesired mutual interactions between the virus liquid components or by interactions of these liquid components with one of the applied auxiliary chemicals.

For example people skilled in the art will certainly not exclude that the added $\beta$-propiolactone would be inactivating one of the virus components too much by acting for a too long period, while such period is necessary of the inactivation of the other component.

The application of the combined vaccine according to the invention is advantageous with respect to the prior art vaccines prepared by mixing of composing vaccines on the spot, in that a smaller volume (e.g. 0.5 cm³ instead of 1 cm³) is necessary for administration, thus causing less local irritation. Moreover a smaller amount of chemicals foreign to the body needs to be administered. Furthermore, problems mentioned hereinabove caused by mixing and introduction of foreign infectious matter are avoided.

The thus prepared vaccines are usually administered to 10 to 20 weeks old birds before the lay.

The invention further relates to a process for preparing a suitable ALV-containing starting liquid which comprises cultivating an adeno-like virus in duck eggs or on a culture of duck embryo fibroblasts e.g. a monolayer rolling bottle culture or a culture of cells attached to solid inert carriers.

According to a preferred embodiment of the invention a mono-layer rolling bottle culture of duck's embryo fibroblasts is formed by suspending the starting cells in a culture medium consisting of at least 70 to 90 parts of Eagle's medium and optionally 5 to 15 parts of calf serum, a 0.22% sodium bicarbonate solution and 1 to 5 parts of a solution or a suspension of one or more antibiotics such as penicillin G, streptomycin and natamycin, and the culture liquid is separated when the cell culture is nearly closed. The cell culture is infected with virus suspension, incubated at 37°C during 0.5 to 2 hours to attach the virus and followed by addition of maintenance medium consisting of Eagle's medium with 2 to 10% of calf serum, 2—10 parts of tryptose phosphate broth (30 g/l), 10—20 parts bovine amnion fluid, and the additives indicated herebefore, and harvesting the virus-containing medium and cells 0.5 to 2 days after the CPE appearance.

The cell culture is inoculated with an ALV suspension having an activity of $\geq 30 \times 10^6$ TCID 50/rolling culture bottle. In this connection is has been found that cell suspension cultures were not suitable for the process discussed herebefore.

For the vaccine preparation a passaged virus-strain 127, as deposited with the Collection d'Institut Pasteur, Paris, under no. I.090, is preferably used and this strain is preferably cultivated in duck eggs.

It is true that Luxembourg Patent Specification No. 79154 generally discloses the preparation of vaccines against the "egg drop syndrome", but the presently proposed preferred production method on a commercial scale for these vaccine liquids of acceptable high titers is not described in any way or even suggested.

In this connection it is pointed to page 2, lines 28 to 30 of the said Luxembourg patent specification, disclosing clearly that the virus may be cultivated on chicken embryo fibroblasts. However it was found that such method certainly did not lead to the desired, economically required results. Page 6 lines 29 to 35 disclose that a culture of embryonal chicken liver cells is used, which however doesn't lead to the desired economically required results either, since cultivation on a commercial scale of embryonal liver cells is still considered a very difficult and thus expensive production method.

The invention is illustrated by the following examples.

Example 1
a) Cultivation of adeno-like virus (passaged strain 127).

In (Belco) rolling culture bottles cell cultures (DEF) are made from duck eggs which were brooded for 12 to 13 days, in Eagle 10% calf serum medium containing 0.22% by weight of $NaHCO_3$ and 2% by volume of an antibiotic solution containing penicillin G, streptocycin and natamycin.

The medium is sucked off when the cell cultures are nearly closed. Then the cultures are inoculated with a suspension of the passaged virus 127 strain, incubated at 37°C for 1 hour and filled up with Eagle's maintenance medium containing 5% of calf serum, 10% by volume of tryptose phosphate broth (30 g/l), 10% by volume of bovine amnion fluid, 0.22% by weight of $NaHCO_3$ and 2% by volume of the above-mentioned antibiotic solution.

The ALV-containing medium and the cells are harvested one day after a CPE appearance and the cells nearly coming off the glass wall. The virus suspension is frozen at −20°C and is stored for vaccine production. The HA titre and the $TCID_{50}$ of this suspension are estimated.

b$_1$) Cultivation of NDV.
SPF eggs which were brooded for 11 days were inoculated with L/Z 258 P virus. The culture was incubated at 37°C for 72 hours and the AAF (Amnion Allantoic Fluid) was harvested. The AAF contains then about $10^{10}$ $EID_{50}$/ml. The virus suspension is frozen at −20°C or lower and is stored for vaccine production.

b$_2$) SPF eggs which were brooded for 11 days were inoculated with Herts 33/56 seed virus. After incubation at 37°C for 24 to 48 hours the AAF is harvested and contains then about $10^{10}$ $EID_{50}$/ml. The virus suspension is frozen at −20°C or lower and is stored for vaccine production.

c) Treatment of virus suspension.
The frozen virus suspensions are defrosted and inactivated with 0.05 to 0.25% of $\beta$-propiolactone in a water bath at 37°C during 90 minutes. The suspensions are stored overnight at +4°C. The inactivation is controlled by observing the formation of CPE's on DEF or CEF followed by a hemabsorption test. Moreover prebrooded SPF chicken eggs are incubated with the inactivated virus fluid and are subsequently controlled.

The virus suspensions are homogenised with an Ultra Turrax homogenizer. If desired the suspension is diluted with PBS+0.3% of formaline, depending on the $EID_{50}$ of the AAF

and HA titre of the adeno like virus suspension. The NDV-AAF and the adeno like virus suspension are mixed thereafter in a ratio of 6:4 and used for emulsion preparation.

d) Preparation of the emulsion.
The virus suspensions are mixed with the oil phase in a ratio of 6.5 parts of oil to 3.5 parts of virus liquid and emulgated in such a way that the average drop size of the aqueous phase is about 0.05—0.5 $\mu$, e.g. by means of injection of the virus suspension into the oil phase with simultaneous homogenizing with an Ultra Turrax homogenizer.

The oily phase is composed as follows:

|  | by volume |
|---|---|
| Marcol® 52 (white paraffinic Esso oil) | 93.6% |
| Arlacel® A or Arlacel 80® or Span 80® (mannite monooleate) | 6.0% |
| Tween® 80 (polyoxyethylene-20-sorbitan monooleate) | 0.4% |

The components of the oily phase are separately heated to 110°C in an autoclave or sterily filtrated as a mixture.

A stable emulsion is obtained, tested by:
1) pipetting directly after emulsifying drops of emulsion onto an aqueous surface, whereby the drops do not spread out but remain intact;
2) allowing to stand at 37°C for 4 weeks, whereby an aqueous phase does not separate.

The final concentrations of the thus prepared emulsions are: 21% NDV containing buffered liquid and 14% ALV 127 containing buffered liquid.

The vaccine obtained is used in a dosage of 0.5 ml per animal intramuscularly in the breast or leg muscles or subcutaneously in the neck. Eight days after vaccination hemagglutination inhibiting anti-compounds are observable.

Example 2
The process of Example 1 is repeated, however, the following changes were made:
a) Instead of the preparation of the ALV according to item a) of example 1, an ALV containing liquid is prepared by inoculating duck eggs, which were previously brooded during 9 days, with the passaged 127 virus strain. After 7 days incubation at 37°C the AAF (amnion allantoic fluid) is harvested. The AAF has then a HA titer of about 1:1024. The virus suspension is subsequently frozen at −20°C and stored for vaccine production.
b) After treatment of the NDV suspension as described under c) of example 1, and inactivation of the ALV suspension with 0.02 to 0.5% formaldehyde during 20 hours at 22°C, the NDV- and the ALV-liquids are mixed in a ratio of 4:1 and used for the preparation of the emulsion as described under d) of example 1.

Example 3

   a) An ALV containing liquid is prepared according to the corresponding step of example 2.

   b) An NDV containing liquid is prepared according to the corresponding step of example 1.

   c) The treatment of the NDV suspension is carried out in the same way as in example 1 and the treatment of the ALV suspension is carried out as in example 2.

   The treated NDV-AAF and ALV-AAF are mixed thereafter in a proportion of 5:1.

   d) The combined virus suspensions are added to and mixed with an oil phase in the proportion of 6.5 parts of oil phase: 3.5 parts of virus containing liquid and an emulsion is prepared by passing it through a colloid mill.

   The applied oil phase has the following composition:

| | |
|---|---|
| Marcol® 52 | 91.4 vol% |
| Arlacel A® or Arlacel® 80 or span® 80 | 8.0 vol% |
| Tween® 80 | 0.6 vol% |

Example 4

   An ALV containing liquid and a NDV containing liquid are prepared according to the respective corresponding steps of example 1. After treatment of the virus suspensions as in c) of example 1, the NDV-AAF and the ALV containing liquid are mixed in a proportion of 4:3 and an emulsion is prepared in the same way as under d) of example 1. However the oil phase and the combined virus containing liquids are mixed now in a proportion of 6:4, while the used oil phase is that one used in example 1 under d).

Example 5

   The combined vaccine, prepared according to example 4, is tested in the following way.

   Not previously vaccinated SPF chickens, being 8 weeks old, are vaccinated with the combined vaccine. The found HAI (hemagglutinating inhibition)-titers resp. the number of HAI-units in the sera coming from the vaccinated animals do not significantly deviate from the antibody levels, found with animals, which were vaccinated with the corresponding single vaccines of a comparable composition under the same conditions.

   The progress curves so obtained by plotting the found HAI-units and HAI-levels respectively expressed in the form of $2^x$ on a logarithmic scale against the time are shown in the annexed figure.

   As to the NDV-HAI-units, significantly higher values were found with animals, which had previously been vaccinated with live NDV vaccine (as is usually done in the field).

   By such animals e.g. about $10^{16.0}$—$10^{17.0}$ NDV-HAI-units are detected in the period of 4 to 8 weeks after vaccination with the combined NDV-ALV vaccine.

**Claims for the Contracting States: CH, DE, GB, IT, NL, SE**

1. A hand ready combined inactivated vaccine against Newcastle disease and egg production drop caused by adeno like viruses, characterized in that it contains an aqueous phase, formed by an inactivated Newcastle disease virus containing liquid with a titre of at least $10^{9.8}$ EID 50/ml and an inactivated adeno like virus containing liquid with a titre of at least $10^2$ HA units/ml obtained by cultivation of an in duck's eggs or in duck's embryo fibroblasts passaged virus strain 127 in duck's eggs or in a cell culture of duck's embryo fibroblasts, whereby the volume ratio between the Newcastle disease containing liquid and the adeno like virus containing liquid can vary from 3:4 to 5:1, and an oil phase comprising as essential components at least one of the components selected from the group consisting of light paraffinic mineral oils, vegetable oils and naphthenic mineral oils and at least one emulsifier selected from the group consisting of esters and ether-esters of alkylene oxide adducts and/or hexahydric alcohols with fatty acids of 10—20 carbon atoms whereby the volume ratio between the aqueous phase formed by both virus liquids and the oil phase can vary from 7:13 to 3:7.

2. Combined inactivated vaccine according to claim 1, characterized in that the volume ratio of the buffered Newcastle disease virus containing liquid and the buffered adeno-like virus containing liquid is 3:2.

3. Combined inactivated vaccine according to claim 1, characterized in that a passaged virus strain 127, deposited under I.090 with the Collection d'Institut Pasteur, Paris, is used.

4. Combined inactivated vaccine according to claim 1, characterized in that a Newcastle disease virus strain L/Z 258 P, deposited under I.091 with the Collection d'Institut Pasteur, Paris, is used.

5. Combined inactivated vaccine according to claim 1, characterized in that the oil phase contains 2 to 20% by weight of an emulsifier and preferably 2 to 15% by weight of di-anhydromannite monooleate or sorbitan monooleate and 0.2 to 4% of polyoxyethylene 20 sorbitan monooleate.

6. Process for the preparation of a combined inactivated vaccine ready for administration, active against Newcastle disease and egg production drops caused by adeno-like viruses, characterized in that an NDV-containing liquid with a titre of at least $10^{9.8}$ EID 50/ml is inactivated, is optionally homogenized, and together with an ALV-containing liquid with a titre of at least $10^2$ HA units/ml obtained by cultivation of an in duck's eggs or in duck's embryo fibroblasts passaged virus strain 127 in duck's eggs or in a cell culture of duck's embryo fibroblasts and previously inactivated and optionally homogenized, whereby the volume ratio between the Newcastle disease containing liquid and the adeno like virus containing liquid may vary from 3:4 to 5:1, is added to an oil phase containing as essential components, at least one of the components selected from the group consisting of light paraffinic mineral oils, vegetable oils and naphtenic mineral oils and at least one emulsifier selected from the group consisting of esters and ether esters of alkylene oxide adducts and/or hexahydric alcohols with fatty acids of 10—20 carbon atoms whereby the volume ratio between the aqueous phase formed by both virus liquids and the oil phase may vary from 7:13 to 3:7, while optionally a preserving agent is added to the aqueous phase.

7. Process according to claim 6, characterized in that as inactivator for the ALV liquid component formaldehyde is used.

8. Process according to claim 6, characterized in that there is started from a NDV liquid having a titre of $10^{9.8}$ $EID_{50}$/ml to $10^{10.5}$ $EID_{50}$/ml.

9. Process according to claim 6, characterized in that there is started from a NDV-liquid having a titre of $\geqslant 10^{10}$ $EID_{50}$/ml.

10. Process according to claim 6, characterized in that an adeno-like virus is cultivated in a monolayer rolling bottle culture or a culture of cells attached to solid inert carriers, of duck's embryo fibroblasts.

11. Process according to claim 10, characterized in that the monolayer cell culture is formed by suspending the starting cells in a culture medium consisting of at least 70 to 90 parts of Eagle's medium, to which are optionally added 5 to 15 parts of calf serum, 0.22% by weight sodium bicarbonate solution and 1 to 5 parts of a solution or a suspension of one or more antibiotics, such as penicillin G, streptomycin and natamycin, the culture medium is separated when the cell culture is nearly closed, is inoculated with the virus suspension, is incubated at 37°C for to 0.5 to 2 hours to attach the virus and maintenance medium, consisting of at least Eagle's medium with 2 to 10% of calf serum, 2—10 parts of tryptose phosphate broth (30 g/l), 10—20 parts bovine amnion liquid and the remaining abovementioned additives, is added, followed by harvesting the virus containing medium and the cells 0.5 to 2 days after the CPE appearance.

12. Process according to claim 11, characterized in that the cell culture is inoculated with an ALV suspension having an activity of $\geqslant 30 \times 10^6$ $TCID_{50}$/rolling culture bottle.

13. Passaged adeno like virus strain 127, deposited under I.090 with the Collection d'Institut Pasteur, Paris.

14. Newcastle disease virus strain L/Z 258 P, deposited under I.091 with the Collection d'Institut Pasteur, Paris.

## Claims for the Contracting State: AT

1. Process for the preparation of a combined inactivated vaccine ready for administration, active against Newcastle disease and egg production drops caused by adeno-like viruses, characterized in that an NDV-containing liquid with a titre of at least $10^{9.8}$ $EID_{50}$/ml is inactivated, is optionally homogenized, and together with an ALV-containing liquid with a titre of at least $10^2$ HA-units/ml obtained by cultivation of an in duck's eggs or in a duck's embryo fibroblasts passaged virus strain 127 in duck's eggs or in a cell culture of duck's embryo fibroblasts and previously inactivated and optionally homogenized, whereby the volume ratio between the Newcastle disease containing liquid and the adeno like virus containing liquid may vary from 3:4 to 5:1, is added to an oil phase containing as essential components, at least one of the components selected from the group consisting of light paraffinic mineral oils, vegetable oils and naphthenic mineral oils and at least one emulsifier selected from the group consisting of esters and ether-esters of alkylene oxyde adducts and/or hexahydric alcohols with fatty acids of 10—20 carbon atoms whereby the volume ratio between the aqueous phase formed by both virus liquids and the oil phase can vary from 7:13 to 3:7, while optionally a preserving agent is added to the aqueous phase.

2. Process according to claim 1, characterized in that the volume ratio of the buffered Newcastle disease virus containing liquid and the buffered adeno-like virus containing liquid is 3:2.

3. Process according to claim 1, characterized in that the oil phase contains 2 to 20% by weight of an emulsifier and preferably 2 to 15% by weight of di-anhydromannite monooleate or sorbitan monooleate and 0.2 to 4% of polyoxyethylene 20 sorbitan monooleate.

4. Process according to claim 1, characterized in that as an inactivator for the NDV liquid component bêta-propiolactone is used, optionally in combination with a stabiliser.

5. Process according to claim 4, characterized in that bêta-propiolactone is applied in a concentration of 0.05 to 0.25% by weight (based on the aqueous phase weight) to the buffered virus containing liquid.

6. Process according to claim 1, characterized in that as inactivator for the ALV liquid component formaldehyde is used.

7. Process according to claim 1, characterized in that there is started from a NDV liquid having a titre of $10^{9.8}$ $EID_{50}$/ml to $10^{10.5}$ $EID_{50}$/ml.

8. Process according to claim 1, characterized in that the cell culture is a monolayer rolling bottle culture or a culture of cells attached to solid inert carriers, of duck's embryo fibroblasts.

9. Process according to claim 8, characterized in that the monolayer cell culture is formed by suspending the starting cells in a culture medium consisting of at least 70 to 90 parts of Eagle's medium, to which are optionally added 5 to 15 parts of calf serum, 0.22% by weight sodium bicarbonate solution and 1 to 5 parts of a solution or a suspension of one or more antibiotics, such as penicillin G, streptomycin and natamycin, the culture medium is separated when the cell culture is nearly closed, is inoculated with the virus suspension, is incubated at 37°C for 0.5 to 2 hours to attach the virus and maintenance medium, consisting of at least Eagle's medium with 2 to 10% of calf serum, 2—10 parts of tryptose phosphate broth (30 g/l), 10—20 parts bovine amnion liquid and the remaining abovementioned additives, is added, followed by harvesting the virus containing medium and the cells 0.5 to 2 days after the CPE appearance.

10. Process according to claim 1, characterized in that the cell culture is inoculated with an ALV suspension having an activity of $\geqslant 30 \times 10^6$ TCID$_{50}$/rolling culture bottle.

11. Process according to claim 1, characterized in that a passaged virus strain 127, deposited under I.090 with the Collection d'Institut Pasteur, Paris, is used.

12. Process according to claim 1, characterized in that a Newcastle disease virus strain L/Z 258 P, deposited under I.091 with the Collection d'Institut Pasteur, Paris, is used.

**Patentansprüche für die Vertragsstaaten: CH, DE, GB, IT, NL, SE**

1. Handfertige, kombinierte, inaktivierte Vakzine gegen Newcastle Disease und ein durch Adeno-ähnliche Viren bewirktes Absinken der Eiproduktion, dadurch gekennzeichnet, daß sie eine wässerige Phase, gebildet von einer inaktiviertes Newcastle Disease-Virus enthaltenden Flüssigkeit mit einem Titer von mindestens 10$^{9,8}$ EID$_{50}$/ml und einer inaktiviertes Adeno-ähnliches Virus enthaltenden Flüssigkeit mit einem Titer von mindestens 10$^2$ HA-Einheiten/ml, erhalten durch Züchten eines in Enteneiern oder in Entenembryofibroblasten passagierten Virusstammes 127 in Enteneiern oder in einer Zellkultur von Entenembryofibroblasten, wobei das Volumsverhältnis zwischen der Newcastle Disease-Virus enthaltenden Flüssigkeit und der das Adeno-ähnliche Virus enthaltenden Flüssigkeit von 3:4 bis 5:1 schwanken kann, und eine Ölphase enthält, die als wesentliche Komponenten mindestens eine der Komponenten ausgewählt aus der Gruppe bestehend aus Leichtparaffinmineralölen, pflanzlichen Ölen und naphthenischen Mineralölen, und mindestens einen Emulgator, ausgewählt aus der Gruppe bestehend aus Estern und Äther-Estern von Alkylenoxidaddukten und/oder 6-wertigen Alkoholen mit Fettsäuren von 10 bis 20 Kohlenstoffatomen, enthält, wobei das Volumsverhältnis zwischen der aus beiden Virusflüssigkeiten gebildeten wässerigen Phase und der Ölphase von 7:13 bis 3:7 betragen kann.

2. Kombinierte, inaktivierte Vakzine nach Anspruch 1, dadurch gekennzeichnet, daß das Volumsverhältnis der gepufferten, Newcastle Disease-Virus enthaltenden Flüssigkeit zu der gepufferten, Adeno-ähnliches Virus enthaltenden Flüssigkeit 3:2 beträgt.

3. Kombinierte, inaktivierte Vakzine nach Anspruch 1, dadurch gekennzeichnet, daß ein passagierter Virusstamm 127, hinterlegt unter I.090 in der Collection d'Institut Pasteur, Paris, verwendet wird.

4. Kombinierte, inaktivierte Vakzine nach Anspruch 1, dadurch gekennzeichnet, daß ein Newcastle Disease-Virusstamm L/Z 258 P, hinterlegt unter I.091 in der Collection d'Institut Pasteur, Paris, verwendet wird.

5. Kombinierte, inaktivierte Vakzine nach Anspruch 1, dadurch gekennzeichnet, daß die Ölphase 2 bis 20 Gew.-% eines Emulgators und vorzugsweise 2 bis 15 Gew.-% Di-anhydromannitmonooleat oder Sorbitanmonooleat und 0,2 bis 4% Polyoxyäthylen-20-sorbitanmonooleat enthält.

6. Verfahren zur Herstellung einer kombinierten, inaktivierten Vakzine, die verabreichungsfertig und gegen Newcastle Disease und gegen ein durch Adeno-ähnliche Viren verursachtes Absinken der Eiproduktion aktiv ist, dadurch gekennzeichnet, daß eine NDV enthaltende Flüssigkeit mit einem Titer von mindestens 10$^{9,8}$ EID$_{50}$/ml inaktiviert und gewünschtenfalls homogenisiert wird und zusammen mit einer ALV enthaltenden Flüssigkeit mit einem Titer von mindestens 10$^2$ HA-Einheiten/ml, erhalten durch Züchten eines in Enteneiern oder in Entenembryofibroblasten passagierten Virusstammes 127 in Enteneiern oder in einer Zellkultur von Entenembryofibroblasten und vorher inaktiviert und gewünschtenfalls homogenisiert, wobei das Volumsverhältnis zwischen der Newcastle Disease-Virus enthaltenden Flüssigkeit und der Adeno-ähnliches Virus enthaltenden Flüssigkeit von 3:4 bis 5:1 schwanken kann, einer Ölphase zugesetzt wird, die als wesentliche Komponenten mindestens eine der Komponenten ausgewählt aus der Gruppe bestehend aus Leichtparaffinmineralölen, pflanzlichen Ölen und naphthenischen Mineralölen, und mindestens einen Emulgator, ausgewählt aus der Gruppe bestehend aus Estern und Äther-Estern von Alkylenoxidaddukten und/oder 6-wertigen Alkoholen mit Fettsäuren von 10 bis 20 Kohlenstoffatomen, enthält, wobei das Volumsverhältnis zwischen der aus beiden Virusflüssigkeiten gebildeten wässerigen Phase und der Ölphase von 7:13 bis 3:7 schwanken kann, und wobei gewünschtenfalls der wässerigen Phase ein Konservierungsmittel zugesetzt wird.

7. Verfahren nach Anspruch 6, dadurch gekennzeichnet, daß als Inaktivator für die ALV-Flüssigkeitskomponente Formaldehyd verwendet wird.

8. Verfahren nach Anspruch 6, dadurch gekennzeichnet, daß von einer NDV-Flüssigkeit mit einem Titer von $10^{9,8}$ $EID_{50}$/ml bis $10^{10,5}$ $EID_{50}$/ml ausgegangen wird.

9. Verfahren nach Anspruch 6, dadurch gekennzeichnet, daß von einer NDV-Flüssigkeit mit einem Titer von $\geqslant 10^{10}$ $EID_{50}$/ml ausgegangen wird.

10. Verfahren nach Anspruch 6, dadurch gekennzeichnet, daß ein Adeno-ähnliches Virus in einer Monolayer-Rollkultur oder einer Kultur von an feste, inerte Träger gebundenen Zellen oder in Entenembryofibroblasten gezüchtet wird.

11. Verfahren nach Anspruch 10, dadurch gekennzeichnet, daß die Monolayer-Zellkultur dadurch gebildet wird, daß die Ausgangszellen in einem Kulturmedium, bestehend aus mindestens 70 bis 90 Teilen Eagle's Medium, dem gewünschtenfalls 5 bis 15 Teile Kalbsserum, 0,22 Gew.-% Natriumbicarbonatlösung und 1 bis 5 Teile einer Lösung oder einer Suspension von einem oder mehreren Antibiotika, wie Penicillin G, Streptomycin und Natamycin, zugesetzt sind, suspendiert werden, das Kulturmedium abgetrennt wird, wenn die Zellkultur fast geschlossen ist, mit der Virus-suspension angeimpft, bei 37°C 0,5 bis 2 h inkubiert wird, um das Virus anzuheften, und Aufrechterhaltungsmedium, bestehend zumindest aus Eagle's Medium mit 2 bis 10% Kalbsserum, 2 bis 10 Teilen Tryptosephosphatbrühe (30 g/l), 10 bis 20 Teilen Rinderamnionflüssigkeit und den weiteren oben erwähnten Zusatzstoffen, zugefügt wird, worauf das Virus enthaltende Medium und die Zellen 0,5 bis 2 Tage nach dem Auftreten des CPE geerntet werden.

12. Verfahren nach Anspruch 11, dadurch gekennzeichnet, daß die Zellkultur mit einer ALV-Suspension mit einer Aktivität von $\geqslant 30 \times 10^6$ $TCID_{50}$/Rollkultur angeimpft wird.

13. Passagierter, Adeno-ähnlicher Virus-Stamm 127, hinterlegt unter I.090 in der Collection d'Institut Pasteur, Paris.

14. Newcastle Disease-Virus Stamm L/Z 258 P, hinterlegt unter I.091 in der Collection d'Institut Pasteur, Paris.

**Patentansprüche für den Vertragsstaat AT:**

1. Verfahren zur Herstellung einer kombinierten, inaktivierten Vakzine, die verabreichungsfertig und gegen Newcastle Disease und gegen ein durch Adeno-ähnliche Viren verursachtes Absinken der Eiproduktion aktiv ist, dadurch gekennzeichnet, daß eine NDV enthaltende Flüssigkeit mit einem Titer von mindestens $10^{9,8}$ $EID_{50}$/ml inaktiviert und gewünschtenfalls homogenisiert wird und zusammen mit einer ALV enthaltenden Flüssigkeit mit einem Titer von mindestens $10^2$ HA-Einheiten/ml, erhalten durch Züchten eines in Enteneiern oder in Entenembryofibroblasten passagierten Virusstammes 127 in Enteneiern oder in einer Zellkultur von Entenembryofibroblasten und vorher inaktiviert und gewünschtenfalls homogenisiert, wobei das Volumsverhältnis zwischen der Newcastle Disease-Virus enthaltenden Flüssigkeit und der Adeno-ähnliches Virus enthaltenden Flüssigkeit von 3:4 bis 5:1 schwanken kann, einer Ölphase zugesetzt wird, die als wesentliche Komponenten mindestens eine der Komponenten ausgewählt aus der Gruppe bestehend aus Leichtparaffinmineralölen, pflanzlichen Ölen und naphthenischen Mineralölen, und mindestens einen Emulgator, ausgewählt aus der Gruppe bestehend aus Estern und Äther-Estern von Alkylenoxidaddukten und/oder 6-wertigen Alkoholen mit Fettsäuren von 10 bis 20 Kohlenstoffatomen, enthält, wobei das Volumsverhältnis zwischen der aus beiden Virusflüssigkeiten gebildeten wässerigen Phase und der Ölphase von 7:13 bis 3:7 schwanken kann, und wobei gewünschtenfalls der wässerigen Phase ein Konservierungsmittel zugesetzt wird.

2. Verfahren nach Anspruch 1, dadurch gekennzeichnet, daß das Volumsverhältnis der gepufferten, Newcastle Disease-Virus enthaltenden Flüssigkeit zu der gepufferten, Adeno-ähnliches Virus enthaltenden Flüssigkeit 3:2 beträgt.

3. Verfahren nach Anspruch 1, dadurch gekennzeichnet, daß die Ölphase 2 bis 20 Gew.-% eines Emulgators und vorzugsweise 2 bis 15 Gew.-% Di-anhydromannitmonooleat oder Sorbitanmonooleat und 0,2 bis 4% Polyoxyäthylen-20-sorbitan-monooleat enthält.

4. Verfahren nach Anspruch 1, dadurch gekennzeichnet, daß als Inaktivator für die NDV-Flüssigkeitskomponente $\beta$-Propiolacton, gewünschtenfalls in Kombination mit einem Stabilisator verwendet wird.

5. Verfahren nach Anspruch 4, dadurch gekennzeichnet, daß das $\beta$-Propiolacton in einer Konzentration von 0,05 bis 0,25 Gew.-% (bezogen auf das Gewicht der wässerigen Phase) der gepufferten, Virus enthaltenden Flüssigkeit zugesetzt wird.

6. Verfahren nach Anspruch 1, dadurch gekennzeichnet, daß als Inaktivator für die ALV-Flüssigkeitskomponente Formaldehyd verwendet wird.

7. Verfahren nach Anspruch 1, dadurch gekennzeichnet, daß von einer NDV-Flüssigkeit mit einem Titer von $10^{9,8}$ $EID_{50}$/ml bis $10^{10,5}$ $EID_{50}$/ml ausgegangen wird.

8. Verfahren nach Anspruch 1, dadurch gekennzeichnet, daß die Zellkultur eine Monolayer-Rollkultur oder eine Kultur von an feste, inerte Träger gebundenen Zellen oder von Entenembryofibroblasten ist.

9. Verfahren nach Anspruch 8, dadurch gekennzeichnet, daß die Monolayer-Zellkultur dadurch gebildet wird, daß die Ausgangszellen in einem Kulturmedium, bestehend aus mindestens 70 bis 90 Teilen Eagle's Medium, dem gewünschtenfalls 5 bis 15 Teile Kalbsserum, 0,22 Gew.-% Natriumbicarbonatlösung und 1 bis 5 Teile einer Lösung oder einer Suspension von einem oder mehreren Antibiotika, wie Penicillin G, Streptomycin und Natamycin, zugesetzt sind, suspendiert werden, das Kulturmedium abgetrennt wird, wenn die Zellkultur fast geschlossen ist, mit der Virussuspension angeimpft, bei 37°C 0,5 bis 2 h inkubiert wird, um das Virus anzuheften, und Aufrechterhaltungsmedium, bestehend zumindest aus Eagle's Medium mit 2 bis 10% Kalbsserum, 2 bis 10 Teilen Tryptosephosphatbrühe (30 g/l), 10 bis 20 Teilen Rinderamnionflüssigkeit und den weiteren oben erwähnten Zusatzstoffen, zugefügt wird, worauf das Virus enthaltende Medium und die Zellen 0,5 bis 2 Tage nach dem Auftreten des CPE geerntet werden.

10. Verfahren nach Anspruch 1, dadurch gekennzeichnet, daß die Zellkultur mit einer ALV-Suspension mit einer Aktivität von $\geqslant 30 \times 10^6$ TCID$_{50}$/Rollkultur angeimpft wird.

11. Verfahren nach Anspruch 1, dadurch gekennzeichnet, daß ein passagierter Virus-Stamm 127, hinterlegt unter I.090 in der Collection d'Institut Pasteur, Paris, verwendet wird.

12. Verfahren nach Anspruch 1, dadurch gekennzeichnet, daß ein Newcastle Disease-Virus-Stamm L/Z 258 P, hinterlegt unter I.091 in der Collection d'Institut Pasteur, Paris, verwendet wird.

**Revendications pour les Etats Contractants: CH, DE, GB, IT, NL, SE**

1. Vaccin inactivé combiné prêt à l'administration contre la maladie de Newcastle et la baisse de la ponte provoquée par des virus du genre adéno, caractérisé en ce qu'il contient une phase aqueuse formée par un liquide contenant du virus inactivé de la maladie de Newcastle ayant un titre d'au moins $10^{9,8}$ DI 50 d'embryon par ml et un liquide contenant du virus de genre adéno inactivé ayant un titre d'au moins $10^2$ unités HA par ml obtenu par culture, dans des oeufs de cane ou dans une culture de fibroblastes d'embryon de canard d'une souche 127 du virus ayant subi des passages dans des oeufs de cane ou dans des fibroblastes d'embryon de canard, étant entendu que le rapport volumique entre le liquide contenant du virus de la maladie de Newcastle et le liquide contenant du virus du genre adéno peut varier de 3:4 à 5:1, et une phase huileuse comprenant comme composants essentiels au moins l'un des composants choisis dans la classe formée par les huiles minérales paraffiniques légères, les huiles végétales et les huiles minérales naphténiques et au moins un émulsionnant choisi dans la classe formée par les esters et éthers-esters de produits d'addition d'oxydes d'alcoylène et/ou d'alcools hexahydroxylés avec des acides gras de 10 à 20 atomes de carbone, étant entendu que le rapport volumique entre la phase aqueuse formée par les deux liquides viraux et la phase huileuse peut varier de 7:13 à 3:7.

2. Vaccin inactivé combiné suivant la revendication 1, caractérisé en ce que le rapport volumique du liquide tamponné contenant du virus de la maladie de Newcastle et le liquide tamponné contenant du virus du genre adéno est de 3:2.

3. Vaccin inactivé combiné suivant la revendication 1, caractérisé en ce qu'une souche 127 du virus ayant subi des passages, déposée sous le numéro I.090 à la Collection de l'Institut Pasteur à Paris, a été utilisée.

4. Vaccin inactivé combiné suivant la revendication 1, caractérisé en ce qu'une souche L/Z 258 P du virus de la maladie de Newcastle, déposée sous le numéro I.091 à la Collection de l'Institut Pasteur à Paris, a été utilisée.

5. Vaccin inactivé combiné suivant la revendication 1, caractérisé en ce que la phase huileuse contient 2 à 20% en poids d'un émulsionnant et de préférence 2 à 15% en poids de monomaléate de dianhydromannite ou de monooléate de sorbitan et 0,2 à 4% de monooléate de polyoxyéthylene 20 sorbitan.

6. Procédé de préparation d'un vaccin inactivé combiné prêt à l'administration actif contre la maladie de Newcastle et la baisse de la ponte provoquée par des virus du genre adéno, caractérisé en ce qu'un liquide contenant du virus de la maladie de Newcastle ayant un titre d'au moins $10^{9,8}$ DI$_{50}$ d'embryon par ml est inactivé, est éventuellement homogénéisé et est ajouté conjointement avec un liquide contenant du virus du genre adéno ayant un titre d'au moins $10^2$ unités HA par ml obtenu par culture, dans des oeufs de cane ou dans une culture de fibroblastes d'embryon de canard, d'une souche 127 du virus ayant subi des passages dans des oeufs de cane ou dans des fibroblastes d'embryon de canard et préalablement inactivée et éventuellement homogénéisée, étant entendu que le rapport volumique entre le liquide contenant du virus de la maladie de Newcastle et le liquide contenant du virus du genre adéno peut varier de 3:4 à 5:1, à une phase huileuse contenant comme composants essentiels au moins l'un des composants choisis dans la classe formée par les huiles minérales paraffiniques légères, les huiles végétales et les huiles minérales naphténiques et au moins un émulsionnant choisi dans la classe formée par les esters et éthers-esters de produits d'addition d'oxydes d'alcoylène et/ou d'alcools hexahydroxylés avec des acides gras de 10 à 20 atomes de carbone, étant entendu que le rapport volumique entre la phase aqueuse formée par les deux liquides viraux et la phase huileuse peut varier de 7:13 à 3:7, tandis qu'un agent de conservation est éventuellement ajouté à la phase aqueuse.

7. Procédé suivant la revendication 6, caractérisé en ce que le formaldéhyde est utilisé comme inactivateur pour le liquide contenant du virus du genre adéno.

8. Procédé suivant la revendication 6, caractérisé en ce qu'on part d'un liquide contenant du virus de la maladie de Newcastle ayant un titre de $10^{9.8}$ $DI_{50}$ d'embryon par ml à $10^{10,5}$ $DI_{50}$ d'embryon par ml.

9. Procédé suivant la revendication 6, caractérisé en ce qu'on part d'un liquide contenant du virus de la maladie de Newcastle ayant un titre $\geqslant 10^{10}$ $DI_{50}$ d'embryon par ml.

10. Procédé suivant la revendication 6, caractérisé en ce qu'on cultive un virus du genre adéno dans un flacon de culture roulant en couche monocellulaire ou dans une culture de cellules fixées sur des supports solides inertes de fibroblastes d'embryon de canard.

11. Procédé suivant la revendication 10, caractérisé en ce qu'on forme la culture. en couche monocellulaire en mettant les cellules de départ en suspension dans un milieu de culture consistant en au moins 70 à 90 parties de milieu d'Eagle auxquelles ont été éventuellement ajoutées 5 à 15 parties de sérum de veau, 0,22% en poids de solution de bicarbonate de sodium et 1 à 5 parties d'une solution ou d'une suspension d'un ou plusieurs antibiotiques tels que la pénicilline G, la streptomycine et la natamycine, on sépare le milieu de culture lorsque la culture de cellules est pratiquement confluente, on l'inocule au moyen de la suspension du virus, on la met à incuber à 37°C pendant 0,5 à 2 heures pour fixer le virus et on ajoute du milieu d'entretien consistant en au moins du milieu d'Eagle avec 2 à 10% de sérum de veau, 2 à 10 parties de bouillon au tryptose-phosphate (30 g par litre), 10 à 20 parties de liquide amniotique bovin et, pour le reste, les additifs précités, après quoi on récolte le milieu contenant le virus et les cellules 0,5 à 2 jours après l'apparition de l'effet cytopathogène.

12. Procédé suivant la revendication 11, caractérisé en ce qu'on inocule la culture de cellules au moyen d'une suspension de virus du genre adéno ayant une activité $\geqslant 30 \times 10^6$ $DI_{50}$ de culture de tissu par flacon de culture roulant.

13. Souche 127 de virus du genre adéno ayant subi des passages, déposée sous le numéro I.090 à la Collection de l'Institut Pasteur à Paris.

14. Souche L/Z 258 P du virus de la maladie de Newcastle, déposée sous le numéro I.091 à la Collection de l'Institut Pasteur à Paris.

**Revendications pour l'Etat Contractant: AT**

1. Procédé de préparation d'un vaccin inactivé combiné prêt à l'administration actif contre la maladie de Newcastle et la baisse de la ponte provoquée par des virus du genre adéno, caractérisé en ce qu'un liquide contenant du virus de la maladie de Newcastle ayant un titre d'au moins $10^{9.8}$ $DI_{50}$ d'embryon par ml est inactivé, est éventuellement homogénéisé et est ajouté conjointement avec un liquide contenant du virus du genre adéno ayant un titre d'au moins $10^2$ unités HA par ml obtenu par culture, dans des oeufs de cane ou dans une culture de fibroblastes d'embryon de canard, d'une souche 127 du virus ayant subi des passages dans des oeufs de cane ou dans des fibroblastes d'embryon de canard et préalablement inactivée et éventuellement homogénéisée, étant entendu que le rapport volumique entre le liquide contenant du virus de la maladie de Newcastle et le liquide contenant du virus de genre adéno peut varier de 3:4 à 5:1, à une phase huileuse contenant comme composants essentiels au moins l'un des composants choisis dans la classe formée par les huiles minérales paraffiniques légères, les huiles végétales et les huiles minérales naphténiques et au moins un émulsionnant choisi dans la classe formée par les esters et éthers-esters de produits d'addition d'oxydes d'alcoylène et/ou d'alcools hexahydroxylés avec des acides gras de 10 à 20 atomes de carbone, étant entendu que le rapport volumique entre la phase aqueuse formée par les deux liquides viraux et la phase huileuse peut varier de 7:13 à 3:7, tandis qu'un agent de conservation est éventuellement ajouté à la phase aqueuse.

2. Procédé suivant la revendication 1, caractérisé en ce que le rapport volumique du liquide tamponné contenant du virus de la maladie de Newcastle et du liquide tamponné contenant du virus du genre adéno est de 3:2.

3. Procédé suivant la revendication 1, caractérisé en ce que la phase huileuse contient 2 à 20% en poids d'un émulsionnant et de préférence 2 à 15% en poids de monomaléate de dianhydromannite ou de monooléate de sorbitan et 0,2 à 4% de monooléate de polyoxyéthylène 20 sorbitan.

4. Procédé suivant la revendication 1, caractérisé en ce que la bêta-propiolactone, éventuellement en combinaison avec un stabilisant est utilisée comme inactivateur pour le liquide contenant du virus de maladie de Newcastle.

5. Procédé suivant la revendication 4, caractérisé en ce que la bêta-propiolactone est ajoutée en une concentration de 0,05 à 0,25% en poids (sur base du poids de la phase aqueuse) au liquide tamponné contenant de virus.

6. Procédé suivant la revendication 1, caractérisé en ce que le formaldéhyde est utilisé comme inactivateur pour le liquide contenant du virus du genre adéno.

7. Procédé suivant la revendication 1, caractérisé en ce qu'on part d'un liquide contenant du virus de la maladie de Newcastle ayant un titre de $10^{9.8}$ $DI_{50}$ d'embryon par ml à $10^{10,5}$ $DI_{50}$ d'embryon par ml.

8. Procédé suivant la revendication 1, caractérisé en ce que la culture de cellules est une culture en couche monocellulaire dans un flacon de culture roulant ou une culture de cellules fixées sur des supports solides inertes de fibroblastes d'embryon de canard.

9. Procédé suivant la revendication 8, caractérisé en ce qu'on forme la culture en couche monocellulaire en mettant les cellules de départ en suspension dans un milieu de culture consistant en au moins 70 à 90 parties de milieu d'Eagle auxquelles ont été éventuellement ajoutées 5 à 15 parties de sérum de veau, 0,22% en poids de solution de bicarbonate de sodium et 1 à 5 parties d'une solution ou d'une suspension d'un ou plusieurs antibiotiques tels que la pénicilline G, la streptomycine et la natamycine, on sépare le milieu de culture lorsque la culture de cellules est pratiquement confluente, on l'inocule au moyen de la suspension du virus, on la met à incuber à 37°C pendant 0,5 à 2 heures pour fixer le virus et on ajoute du milieu d'entretien consistant en au moins du milieu d'Eagle avec 2 à 10% de sérum de veau, 2 à 10 parties de bouillon au tryptose-phosphate (30 g par litre), 10 à 20 parties de liquide amniotique bovin et, pour le reste, les additifs précités, après quoi on récolte le milieu contenant le virus et les cellules 0,5 à 2 jours après l'apparition de l'effet cytopathogène.

10. Procédé suivant la revendication 1, caractérisé en ce qu'on inocule la culture de cellules au moyen d'une suspension de virus du genre adéno ayant une activité $\geqslant 30 \times 10^6$ $DI_{50}$ de culture de tissu par flacon de culture roulant.

11. Procédé suivant la revendication 1, caractérisé en ce qu'on utilise une souche 127 du virus ayant subi des passages, déposée sous le numéro I.090 à la Collection de l'Institut Pasteur à Paris.

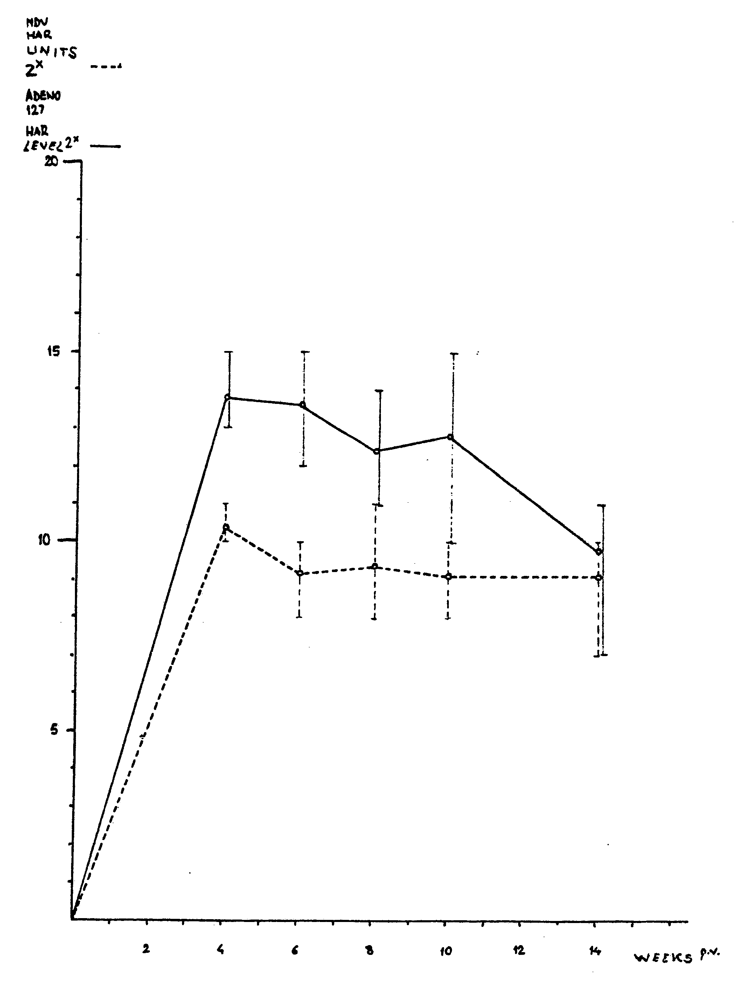

12. Procédé suivant la revendication 1, caractérisé en ce qu'on utilise une souche L/Z 258 P du virus de la maladie de Newcastle, déposée sous le numéro I.091 à la Collection de l'Institut Pasteur à Paris.

# 0 009 277

NDV
HAR
UNITS
$2^x$ ----

ADENO
127
HAR
LEVEL $2^x$ ——

WEEKS P.V.

1